# EUROPEAN PATENT SPECIFICATION

(11) **EP 2 068 873 B1**
(45) Date of publication and mention of the grant of the patent: **09.11.2011**
(21) Application number: 07801393.5
(22) Date of filing: 14.09.2007
(51) Int. Cl.: A61K 31/44, A61P 17/02

(54) **ISONIAZID MEDIATED HEALING OF WOUNDS AND ULCERS**
ISONIAZID-VERMITTELTE HEILUNG VON WUNDEN UND GESCHWÜREN
GUÉRISON DES BLESSURES ET DES ULCÈRES SOUS LA MÉDIATION DE L'ISONIAZIDE

(30) Priority: 14.09.2006 DK 200601187
(43) Date of publication of application: 17.06.2009
(73) Proprietor: Bridge BioResearch Rights (Jersey) Limited, St. Helier Jersey JE2 4UH (GB)
(72) Inventor: WULFF, Trine, 3050 Humlebæk (DK); LANGE, Sven, 3520 Farum (DK); ÅGREN, Magnus, 3050 Humlebæk (DK)
(74) Representative: Høiberg, Susanne
(86) International application number: PCT/DK2007/050128
(87) International publication number: WO 2008/031440

(56) References cited:
- WO-A-01/32218
- WO-A-03/068232
- WO-A-2004/047842
- WO-A-2005/070006
- WO-A-2006/015317
- WO-A-2007/060453
- WO-A-2007/142942
- RO-A2- 69 911
- US-A1- 2005 069 518
- US-A1- 2006 188 471
- US-B1- 6 355 858
- AGAEV, A. YU. ET AL: "Experimental antioxidant therapy of purulent wounds" BYULLETEN EKSPERIMENTAL'NOI BIOLOGII I MEDITSINY , 108(7), 35-7 CODEN: BEBMAE; ISSN: 0365-9615, 1989, XP002477307

## Description

### Field of invention

The present invention relates to the use of isoniazid in the preparation of a pharmaceutical composition for treating (or preventing) skin or mucosa lesions, wounds, ulcers or infarcts in a tissue area with compromised vascularisation in subjects.

### Background of the invention

The process of wound healing may be separated into three phases: an inflammatory phase, a proliferative phase, and a re-modeling phase, which may overlap in time. Wounds that heal normally progress through these phases in an orderly fashion within less than 8 weeks. Wounds that fail to heal are often interrupted in one of the phases, usually continued inflammation or proliferation. Such condition may be further complicated by secondary infections. Wounds are considered chronic if healing has not completed or satisfactory progressed within an 8 week period i.e. if the wound has not contracted by at least 10 % in size, and if healing of the wound is not sustained. Chronic wounds may take years to heal, will re-occur, or never heal at all.

The vast majority of chronic wounds can be classified as vascular ulcers (due to underlying arterial or venous insufficiency), diabetic ulcers, or pressure ulcers; Often the causes of wounds are multifactorial, for example in the diabetic patient who has both arterial insufficiency and peripheral neuropathy.
ischemia is the reduction or abolition of blood supply to a tissue. The associated deficiency of oxygen and nutrients may lead to cell death necrosis in areas of the affected tissue. Ischemic tissue becomes inflamed and factors are released that attract neutrophils. Apart from being active phagocytes, neutrophils also release inflammatory cytokines, reactive oxygen species (ROS), and enzymes that damage cells, inhibit cell proliferation, and thereby inhibit wound closure. Neutrophils remain in chronic wounds for longer than they do in acute wounds, and contribute to the fact that chronic wounds have higher levels of inflammatory cytokines and ROS. Ischemia and the consequences of ischemia is an important factor in the formation and persistence of wounds partly by stalling the wound healing process in the inflammatory phase.

A major cause of chronic wounds is diabetes. A diabetic ulcer is an open sore or wound that most commonly occur on the bottom of the foot (diabetic foot ulcer). People with diabetes can develop diabetic ulcers and using insulin or suffering from other diabetes related diseases increase the risk. Other risk factors include overweight and the use of alcohol and tobacco. According to the American Podiatric Medical Association (APMA) approximately 15 percent of patients with diabetes develop diabetic foot ulcers of which six percent will be hospitalized due to infection or other ulcer-related complication. Diabetes is the leading cause of nontraumatic lower extremity amputations in the United States, and 14 to 24 percent of patients with diabetes who develop a foot ulcer will have an amputation.

Diabetic ulcers develop due to a combination of several factors including neuropathy, poor circulation, irritation (e.g. friction or pressure) and trauma. The condition may be complicated if the patient also suffers from Lower Extremity Peripheral Arterial Disease (LEPAD) or other vascular diseases, which decreases the arterial perfusion to the lower extremities, delays the healing of the wound, and increases the risk of infection. The clinical symptoms of diabetic ulcers can be divided into stages according to different classifications systems.

### The Wagner classification system:

Grade 0 - Skin with prior healed ulcer scars, areas of pressure which are sometimes called pre-ulcerative lesion or the presence of bony deformity which puts pressure on an unguarded point.
Grade I-A - The wound is superficial in nature, with partial- or full-thickness skin involvement but does not include tendon, capsule or bone.
Grade I-B - As above, the wound is superficial in nature, with partial- or full-thickness skin involvement but not including tendon, capsule or bone; however the wound is infected. The definition of this wound implies superficial infection without involvement of underlying structures. If the wound shows signs of significant purulence or fluctuance, further exploration to expose a higher grade classification of infection is in order.
Grade I-C - As above but with vascular compromise.
Grade I-D - As above but with ischemia. Because ischemia is a type of vascular compromise, the distinction between these two grades is often difficult to make.
Grade 2-A - Penetration through the subcutaneous tissue exposing tendon or ligament, but not bone.
Grade 2-B - Penetration through the deep tissues including tendon or ligament and even joint capsule, but not bone.
Grade 2-C - As above 2B, but including ischemia.
Grade 2-D - As above 2C, but including infection.
Grade 3-A - A wound which probes to bone, but neither shows signs of local infection nor systemic infection.
Grade 3-B - A wound which probes to bone, and is infected.
Grade 3-C - A wound which probes to bone, is infected, and is ischemic.
Grade 3-D - A wound which probes to bone, characterized by active infection, ischemic tissues, and exposed bone.
Grade 4 - Gangrene of the forefoot.
Grade 5 - Gangrene of the entire foot.

The University of Texas (UT) classification system (JeffcoateWJ- et al, 1993 Diabet Med 10:676) is a diabetic wound classification system combining grade and stage:

| | 0 | I | II | III |
|---|---|---|---|---|
| A | areas of pressure which are sometimes called pre-ulcerative lesion | superficial ulcer not including tendon, capsula or bone | deep ulcer including tendon, capsula but not bone | deep ulcer including bone and articulation |
| B | infection | infection | infection | infection |
| C | ischemia | ischemia | ischemia | ischemia |
| D | infection+ ischemia | infection+ ischemia | infection+ ischemia | infection+ ischemia |

The University of Texas classification system is easier to use and shows a greater correlation with prediction of ulcer healing when compared with the Wagner classification system (Samson-OO et al (2001) Diabetes Care 24:84).

Pressure ulcers (pressure sores, bed sores, plaster ulcer, decubitus, decubitus ulcers, decubitus sores, ischial tuberosity ulcers) is a skin injury caused by sustained pressure on a bony part of the body (e.g tailbone, buttocks, back of a leg or an arm).

According to the European Pressure Ulcer Advisory Panel pressure ulcers may be classified as:
Grade 1: non-blanchable erythema of intact skin. Discolouration of the skin, warmth, oedema, indurations or hardness may also be used as indicators, particularly on individuals with darker skin.
Grade 2: partial thickness skin loss involving epidermis, dermis, or both. The ulcer is superficial and presents clinically as an abrasion or blister.
Grade 3: full thickness skin loss involving damage to, or necrosis of, subcutaneous tissue that may extend down to, but not through underlying fascia.
Grade 4: extensive destruction, tissue necrosis, or damage to muscle, bone, or supporting structures with or without full thickness skin loss.

The Wagner classification system and the University of Texas classification system developed for diabetic ulcers are also frequently used by clinicians to evaluate pressure ulcers.

Patients with pressure ulcers are typically seniors, bedbound or (wheel)chairbound either permanently or for a prolonged period of time due to a spinal cord injury, surgery or other treatment requiring longer hospital stays, or over weight. Although many factors contribute to the development of pressure ulcers, pressure leading to ischemia is the final common pathway. Pressure ulcers can be very painful, are often difficult and costly to treat. First step in the management of pressure ulcers is to relieve the affected area from the insult causing the condition (pressure and eventual mechanical stress). Second in line is the treatment of the wound. Wound dressings vary with the state of the wound. Early stage of lesions may not require any dressing. Later stage ulcers may be treated with a hydrocolloid occlusive dressing (DuoDerm or similar), which maintains a moist environment to facilitate re-epithelization. For more advanced ulcers, a large variety of treatment options is available. These include wet-to-dry dressings, incorporating isotonic sodium chloride solution or dilute Dakins solution (sodium hypochlorite), Silvadene, Sulfamylon, hydrogels (such as Carrington gel, Intrasite Gel, Granugel, Nu-gel), xerogels (Sorbsan), and vacuum-assisted closure (VAC) sponges.

Pressure ulcers are a serious problem that affects 14% of all acute care clients, 23% of nursing home clients, 25% of rehabilitation hospital clients, 19% of home healthcare clients and 13% of hospital-based hospice agency clients (U.S. Department of Health and Human Services, 1994). The prevalence and incidence of pressure ulcers is increasing steadily. This despite the fact that measures are taken to prevent the development of pressure ulcers including the avoidance of pressure points anywhere on the body surface by use of air and water cushions, mattresses, regular change of position of the body and the limbs, and stimulation of the circulation in the areas at risk. The cost associated with caring for clients with pressure ulcers is staggering. It is estimated that 3% of U.S. healthcare costs are due to pressure ulcer-related diagnoses. The total cost of caring for these wounds is estimated between $3 and $7 billion.

Peripheral Arterial Disease, (PAD), and Lower Extremity Peripheral Arterial Disease (LEPAD) is collectors of all diseases manifesting themselves by the obstruction of large peripheral arteries, which result either from atherosclerotic or inflammatory processes causing lumen narrowing (stenosis), or from thrombus formation (usually associated with underlying atherosclerotic disease). When these conditions arise, there is an increase in vessel resistance that can lead to a reduction in distal perfusion pressure and blood flow leading to ischemia (acute or chronic). Patients are evaluated by clinicians by various means of noninvasive vascular tests such as plethysmography, waveform, Ankle Brachial Indices (Doppler test) and Transcutaneous Oxygen Pressure measurements (TCPO2). On the basis of ABI measures, the prevalence of LEPAD is approximately 3% in people younger than 60 years in the US. The prevalence increases to 20% in people older than 70 years.

Symptoms of the diseases (PAD and LEPAD) encountered on physical examination include pain (claudication) with walking or at rest, decreased or absent distal pulses; bruit over a tightly narrowed artery; hair loss; thickened nails; shiny skin. The most frequent complications associated with the disease are chronic ulcers and gangrene, which may lead to amputation of a toe or a limb. In fact, most ulcers on the foot and leg are caused by underlying vascular insufficiency.

State-of-the-art treatment of chronic wounds address the cause of the condition (e.g ischemia or infection) by means of antibiotic treatment, debridation (e.g. surgical), irrigation, negative pressure wound therapy (NPTW), oxygenation, moist wound healing, removing mechanical stress, and adding cells or administrate growth factors or hormones that promote healing the healing.

The management of chronic wounds is lengthily, difficult and costly due to nursing time and is very sensitive to the development of complications requiring hospitalization. In the US, an estimated 4 million patients are treated for a chronic wound annually, a number expected to rise to more 6 million in 2005. The associated cost of treatment is in excess of 10 billion US$. The number of wounds seen in the EU is more than 3 million per year, expected to increase to more than 5 million wounds in 2005. The increasing prevalence of chronic wounds is a consequence of aging population and the increasing prevalence of lifestyle diseases such as obesity, Type 2 diabetes, and atherosclerosis. Apart from being costly to manage, associated with severe pain and discomfort, chronic wounds are also associated with a social burden due to psychological distress and social isolation.

Accordingly, there is a need for cost-effective treatments of chronic lesions with underlying vascular compromise. This is supported by a recent systematic review concluding that there are no treatments that effectively promote the healing of arterial leg ulcers (Nelson EA and Bradley MD, 2003). Moreover, the conditions are associated with severe pain, discomfort, psychological distress and may lead to social isolation due to immobility.

It has been known for decades that when treating tuberculosis patients with isoniazid coincidental non-ischemic cutaneous lesions healed. The same was true for non-tuberculosis patients treated with isoniazid (Potter and Taylor, 1969). Martyn and Campell (1963) published a case report showing the stimulating effects of isoniazid on the healing of a variety of non-TB chronic wounds. However, the authors concluded that the effect of isoniazid is a vascular phenomenon with little effect on ischemic tissue.

### Summary of invention

The invention enclosed show that isoniazid unexpectedly have a profound stimulation on the healing of ischemic wounds. The present invention provide new efficient treatments of skin lesions, chronic wounds, ulcers or infarcts in a tissue area with compromised vascularisation in a field where such cost-effective therapies are needed.

The invention relates to the use of isoniazid. or pharmaceutically acceptable salts or solvates thereof, for the preparation of a pharmaceutical composition for treating a condition selected from the group consisting of cutaneous lesions, wounds, ulcers and infarcts in a tissue area with compromised vascularisation, in an animal, such as a human subject.

The compound is preferably isoniazid, or pharmaceutical acceptable salts or solvates thereof, and the conditions to be treated is preferably selected from the group consisting of pressure ulcers, diabetic ulcers, arterial ulcers and anastomotic ulcers.

### Description of Drawings

Fig. 1. Insertion of Licox^{®} probe via Venflon catheter to the edge of a non-ischemic wound outside the flap
Fig. 2. Course of healing of non-ischemic (Fig. 2A) and ischemic wounds (2B). Mean values.

### Detailed description of the invention

The present inventors have surprisingly found that cutaneous lesions, wounds, ulcers, and infarcts in a tissue area with compromised vascularisation can be treated by the administration of isoniazid.

Herein disclosed is the use of a compound(s) selected from isoniazid and isoniazid analogs of formula la, 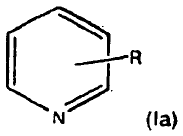 wherein R is -C(O)NHNH₂, -C(O)NHNHC₁₋₆ alkyl, -C(O)NHN=C₁₋₆ alkenyl, -C(O)NHNHC₂₋₅ alkenyl, -C(O)NHC₁₋₆ alkyl, -C(O)NHC₂₋₆ alkenyl, -C(O)NHNHC(O)C₁. ₆alkyl, -NHC(O)C₁₋₆ alkyl, -NHC(O)C₁₋₆ alkenyl, -NHC(O)NH₂, -NHC(O)NHC₁₋₆ alkyl, - NHC(O)NHC₂₋₈ alkenyl, or -COOH, and wherein formula I optionally is further substituted with one or more substituents;
or pharmaceutically acceptable salts, solvates or prodrugs thereof, for the preparation of a pharmaceutical composition for treating a condition selected from the group consisting of cutaneous lesions, wounds, ulcers and infarcts in a tissue area with compromised vascularisation, in an animal, such as a human subject.

The R-group of formula la may be present in the 2-, 3- or 4-position of the pyridine ring. R is preferably present in the 4-position of the pyridine ring. Formula (I b) shows the compound, wherein R is present in the 4-position of the pyridine ring. 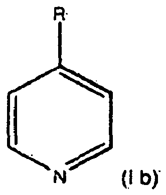 In one compound, R is -C(O)NHNH₂, -C(O)NHNHC₁₋₆ alkyl, -C(O)NHC₁₋₆ alkyl, -C(O)NHNHC(O)C₁₋₆alkyl, or -COOH; and in a preferred compound R is -C(O)NHNH₂, or -COOH. In a more preferred compound R is -C(O)NHNH₂. In a even more preferred compound R is-C(O)NHNH₂ and is in the 4-position of the pyridine ring.

Isoniazid (pyridine-4-carbohydrazide) is a bactericidal agent active against organisms of the genus Mycobacterium, specifically M. tuberculosis, M. bovis and M. kansasii. It is a highly specific agent, ineffective against other microorganisms. Isoniazid is a first-line tuberculosis -medication and has been used for years for the treatment of all forms of tuberculosis (TB) in which organisms are susceptible. Patients treated with isoniazid may show adverse drug reaction including rash, abnormal liver function tests, hepatitis, peripheral neuropathy, mild central nervous system (CNS) effects. The metabolism of isoniazid is primarily hepatic. Isoniazid is acetylated by N -acetyl transferase to N - acetylisoniazid; it is then biotransformed to isonicotinic acid and monoacetylhydrazine, the later is associated with the hepatotoxicity. In a preferred embodiment of the invention, the compound used is isoniazid, or pharmaceutical acceptable salts. Or solvates thereof. Formula (II) gives the structure of isoniazid. 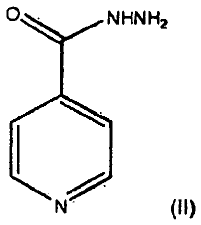 The term "isoniazid analog" refers to any compound with a chemical structure related to isoniazid and bioactivities similar to isoniazid.

The term "C₁₋₆alkyl" means a saturated linear or branched hydrocarbon group including, for example, methyl, ethyl, isopropyl, t-butyl, pentyl, hexyl, and the like.

The term "=C₁₋₆alkenyl" means an unsaturated linear or branched hydrocarbon group with a double-bond to another part of the structure, including, but not limited, to, methylidene (=CH₂), ethylidene (=CHCH₃), propylidene (=CHCH₂CH₃), isopropylidene (=C(CH₂)₂), butylidene (=CHCH₂CH₂CH₃).

The term "C₂₋₆alkenyl" means an unsaturated linear or branched hydrocarbon group with one or more carbon double-bonds, including, but not limited to, ethylene, propylene, isopropylen, butylen, pentylen, hexylen.

As is well understood in this technical area, a large degree of substitution is not only tolerated, but is often advisable. Substitution is anticipated on the pyridine ring and on C₁₋₆alkyl-groups, it any, of the isoniazid analogs to be used in the present invention. The term "substituents" are used to differentiate between the pyridine ring or C₁₋₆alkyl-group of formula (Ia) and formula (I) and further chemical species that may be substituted on to the pyridine ring or C₁₋₆alkyl-group. Non-limiting examples of suitable substituents may be hydrocarbon alkyl substituents, such as methyl, ethyl, propyl, t-butyl, and the like, and further substituents known in the art, such as hydroxy, alkoxy, alkylsulfonyl, halogen, such as e.g. fluoro, iodo and chloro; cyano, nitro, amino, aminoalkyl, carboxyl, aryl, heteroaryl, cycloalkyl, common amino acids etc. It is well-known that these substituents may include further substitution, such for example, hydroxy, alkoxy, alkylsulfonyl, halogen atoms, cyano, nitro, amino, carboxyl, common amini acids etc.

There may be one or more substituents on the isoniazid analogs defined by formula (Ia), such as e.g. 1, 2, 3, or 4 substituents. Preferably formula (Ia) is substituted with 1 or 2 substituents.

The term "aryl" means a mono- or polycyclic aromatic hydrocarbon group.

The term "heteroaryl" means a monovalent aromatic cyclic radical having one to three rings, of four to eight atoms per ring, incorporating one or two heteroatoms (chosen from nitrogen, oxygen, or sulphur) within the ring.

The term "cycloalkyl" means a monovalent saturated carbocyclic radical consisting of one, two or three rings, of three to eight carbons per ring.

The term "common amino acid moiety" means the naturally occurring α-amino acids. unnatural amino acids, substituted β and γ amino acids and their enantiomers. Non-limiting examples are alanine, β-alanine, arginine, asparagine, aspartic acid, cysteine, glutamic acid, glutamine, glycine, histidine, isoleucine, leucine, lysine, methionine, phenylalanine, praline, serine, threonine, tryptophan, tyrosine, valine, 3-hydroxyproline, N-methylphenylalanine, N-methylisoleucine, norvaline, norleucine, ornithine, 2-aminobutyric acid, 2-aminoadipic acid, methionine sulfoxide, methionine sulfone, phenylglycine, o-methyltyrosine, etc.

When the compound to be used in the present invention contain asymmetric carbon atoms, the compound or the pharmaceutical acceptable salts or solvates thereof, may exist as single stereoisomers, racemates; and/or mixtures of enantiomers and/or diastereomers. Where a compound contains an alkenyl, alkenylene, or alkylidene group, geometric cis/trans (or Z/E) isomers are possible; and where the compound contains, for example, a keto or oxime group, tautomeric isomerism ('tautomerism') may occur. It follows that a single compound may exhibit more than one type of isomerism. All such single stereoisomers, racemates, geometric isomers, tautomeric forms and mixtures thereof are intended to be within the scope of the present invention.

The terms "Pharmaceutical composition", "drug", "medicaments" or "agent" refers to any chemical or biological material, compound, or composition capable of inducing a desired therapeutic effect when properly administered to a patient. Some drugs are sold in an inactive form that is converted in vivo into a metabolite with pharmaceutical activity. For purposes of the present invention, the terms "pharmaceutical composition" and "medicament" encompass both the inactive drug and the active metabolite.

The term "pharmaceutically acceptable" means that the substance or composition must be compatible with the other ingredients of a formulation, and not deleterious to the patient.

The terms "treating", "treat" or "treatment" include both preventative (e.g., prophylactic), palliative, and curative treatment, together with a treatment to reduce symptoms.

As used herein, the term "salt" includes, but is not limited to, any possible base or acid addition salts of isoniazid. The acid addition salts are formed from basic compounds, whereas the base addition salts are formed from acidic compounds. All of these forms are within the scope of the present invention. A pharmaceutically acceptable salt of a compound to be used in the present invention may be readily prepared by mixing together solutions of the compound and the desired acid or base, as appropriate. The salt may precipitate from solution and be collected by filtration or may be recovered by evaporation of the solvent. The degree of ionisation in the salt may vary from completely ionised to almost non-ionised. The free acid forms or free base forms of the compounds differ from their respective salt forms somewhat in certain physical properties such as solubitity, crystal structure, hygroscopicity, and the like, but otherwise the salts are equivalent to their respective free acid or free base for purposes of the present invention. Non limiting examples of counter ions for the base additions salts are a metal cation, such as an alkali or alkaline earth metal cation, or an amine, especially an organic amine. Examples of suitable metal cations include sodium cation (Na+), potassium cation (K+), magnesium cation (Mg2+), calcium cation (Ca2+), and the like. Examples of suitable amines are N,N'-dibenzylethylenediamine, chloroprocaine, choline, diethanolamine, dicyclohexylamine, ethylenediamine, N-methylglucamine, and procaine (see, for example, Berge S.M. et al., "Pharmaceutical Salts," J. of Pharma. Sci., 1977;66:1). Suitable acid addition salts are formed from acids which form non-toxic salts. Examples include the acetate, aspartate, benzoate, besylate, bicarbonate/carbonate, bisulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, D- and L-lactate, malate, maleate, malonate, mesylate, methylsulphate, naphthylate, 2-napsylate, nicotinate, nitrate, orotate, oxalate, palmitate, palmoate, phosphate, hydrogen phosphate, dihydrogen phosphate, saccharate, stearate, succinate, sulphate, D- and L- tartrate, tosylate and trifluoroacetate salts.

As used herein, the term "solvate" means a compound of the invention or a salt thereof that further includes a stoichiometric or non-stoichiometric amount of a solvent bound by non-covalent intermolecular forces. Preferred solvents are volatile, non-toxic, and/or acceptable for administration to humans in trace amounts. The solvated forms, including hydrated forms, are equivalent to unsolvated forms and are encompassed within the scope of the present invention.

As used herein, the term "prodrug" means a substance that is transformed *in vivo* to yield a substance of the present invention. The transformation may occur by various mechanisms, such as through hydrolysis in blood. For example, when a compound of the present invention contains a carboxylic acid functional group, a prodrug can comprise an ester formed by the replacement of the hydrogen atom of the acid group with a group including, but not limited to, groups such as for example (C₁-C₆)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N (alkoxycarbonyl)amino)ethyl having from 4 to 10 carbon atoms, 3-phthalidyl, 4 crotonolactonyl, gamma-butyrolacton-4-yl, di-N,N-(C₁-C₂)alkylamino(C₂-C₃)alkyl, carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl. The prodrug can furthermore comprise e.g. an amide formed by the replacement of the hydrogen atom of an acid group with a common amino acid moiety, non-limiting examples of common amino acids are mentioned herein above.

The present invention relates to the treatment of lesion, wounds and ulcers, such as e.g. chronic lesions, chronic cutaneous or mucosa lesions, wounds, ulcers or infarcts with an underlying compromised vascularisation. Chronic lesions with underlying vascular compromise according to the present invention include cutaneous lesions such as wounds, ulcers, in particular pressure ulcers, diabetic ulcers, arterial ulcers and chronic mucosa lesions such as anastomotic ulcer. More particular the invention relates to the treatment of diabetic ulcer, pressure ulcers and arterial ulcers. Preferably the invention relates to the treatment of a human subject.

In a specific-embodiment of the present invention, the condition is selected from the group consisting of pressure ulcers, diabetic ulcers, arterial ulcers and anastomotic ulcers.

The Agency for Health Care Policy and Research (AHCPR) describes a suitable scale for assessing pressure ulcers in: Clinical Practice Guideline No. 3, Pressure ulcers in adults: prediction and prevention; May 1992. The scale is divided in four stages as described below.

Stage I: Nonblanchable erythema of intact skin, the heralding lesion of skin ulceration. In individuals with darker skin, discoloration of the skin, warmth, edema, induration, or hardness may also be indicators.

Stage II: Partial thickness skin loss involving epidermis, dermis, or both. The ulcer is superficial and presents clinically as an abrasion, blister, or shallow crater.

Stage III: Full thickness skin loss involving damage to or necrosis of subcutaneous tissue that may extend down to, but not through, underlying fascia. The ulcer presents clinically as a deep crater with or without undermining of adjacent tissue.

Stage IV: Full thickness skin loss with extensive destruction, tissue necrosis; or damage to muscle, bone, or supporting structures (e.g., tendon, joint capsule). Undermining and sinus tracts also may be associated with Stage IV pressure ulcers The National Pressure Ulcer Advisory Panel (NPUAP) have provided clinical practice guidelines for pressure ulcer care, with the following grading system

| | |
|---|---|
| Stage 1: | Nonblanchable erythema of intact skin |
| Stage 2: | Superficial or partial thickness skin loss Ulcer involves Epidermis or Dermis Skin abrasion Blister |
| Stage 3: | Full thickness skin loss with Subcutaneous damage Ulcer extends down to underlying fascia Presents as deep crater |
| Stage 4: | Full thickness skin loss with extensive destruction Tissue necrosis Damage to muscle, bone, tendon or joint capsule |

The term "compromised vascularisation" refers to a condition where the blood supply is reduced such as in the Grade I-C ulcers according to the Wagner classification system of ulcers.

The term "ischemia" or "Ischemic" refers to a restriction in blood supply with resultant dysfunction or damage of tissue. In a preferred embodiment of the invention, the tissue area with compromised vascularisation is ischemic. Upon suspicion of ischemic leg ulcers, the first-line test is the noninvasive vascular Doppler test which is used to detect evidence of peripheral vascular disease. The Ankle Brachial Index (ABI), obtained by using the Doppler test, is a measure of the fall in blood pressure in the arteries supplying the legs and as such is used to detect evidence of peripheral vascular disease associated with arterial ulcers in particular. ABI is calculated by dividing the systolic blood pressure in the ankle by the higher of the two systolic blood pressures in the arms. An Ankle Brachial Index (ABI) of less than 0.95 is a strong predictive sign of lower-extremity perfusion compromise; an ABI between 0.6 and 0.8 is the borderline of ischemia suggesting that the patient is at risk of developing a nonhealing or slow-healing wound, less than 0.5 indicate severe ischemia, an ABI less than 0.2 is associated with gangrene. Other measures such as toe pressure (TP) less than 30 mmHg is an indicator of LEPAD, and a Transcutaneous Oxygen Pressure measure (TcPO₂) less 40 mmHg is associated with impaired wound healing. Using the Doppler test on patients suffering from diabetes may result in false negative due to poorly compressible blood vessel, which results in falsely elevated ankle pressure. In a preferred embodiment of the invention, the toe pressure (TP) is less than 40 mmHg.

Gangrene is necrosis and subsequent decay of body tissues caused by infection or thrombosis or lack of blood flow. It is usually the result of critically insufficient blood supply sometimes caused by injury and subsequent contamination with bacteria. This condition is most common in the extremities. Gangrene caused by a serious bacterial infection is called wet gangrene. Gangrene caused by lack of circulation in an injured or diseased area is called dry gangrene. In a preferred embodiment of the invention, the condition is not gangrene.

Chronic: Lesions, wounds, ulcers, and infarcts are considered "chronic" if healing has not completed within an 8 week period or if the wounds has not contracted by at least 10 % in size over a period of 8 weeks, and if healing of the wound is not sustained. Thus, chronic reflect a condition where normal healing of the lesion is interrupted or severely impaired. In a preferred embodiment of the invention, the condition is chronic.

Pressure ulcer: Any lesion caused by unrelieved pressure, resulting in damage of underlying tissue. Pressure ulcers usually occur over bony prominences and are graded or staged to classify the degree of tissue damage observed. Bed- and chair-bound individuals or those with impaired ability to reposition should be assessed for additional factors that increase the risk of developing pressure ulcers. Pressure ulcers are evaluated and graded by the clinicians using different grading systems. According to AHCPR's Clinical Practice Guideline No. 3 pressure ulcers may be describe as follows:Stage I pressure ulcers are defined as nonblanchable erythema of intact skinthe heralding lesion of skin ulceration (reactive hyperemia should not be confused with Stage I pressure ulcers). Stage II is defined as partial thickness skin loss involving epidermis and/or dermis; Stage III as full thickness skin loss involving damage or necrosis of subcutaneous tissue that may extend down to, but not through, underlying fascia; and Stage IV as full thickness skin loss with extensive destruction, tissue necrosis or damage to muscle, bone, or supporting structures. Other classification systems often used to grade pressure ulcers include the Wagner classification system and the University of Texas classification system described herein.

Diabetic ulcers: Refer to ulcers, which occur in humans with diabetes mellitus, and are caused by the combination of arterial blockage (sclerosis of minor et major arteries) and nerve damage. Diabetic ulcers arse associated with diabetes mellitus. Although diabetic ulcers may occur on other parts of the body they are more common on the foot. The nerve damage or sensory neuropathy reduces awareness of pressure, heat or injury. Rubbing and pressure on the foot goes unnoticed and causes damage to the skin and subsequent 'neuropathic' ulceration. Signs of peripheral neuropathy include loss of vibratory and position sense, loss of deep tendon reflexes (especially loss of the ankle jerk), trophic ulceration, foot drop, muscle atrophy, and excessive callous formation, especially overlying pressure points such as the heel. The nylon monofilament test helps diagnose the presence of sensory neuropathy. A 10-gauge monofilament nylon is pressed against each specific site of the foot just enough to bend the wire. If the patient does not feel the wire at 4 or more of these 10 sites, the test is positive for neuropathy. General use filaments can be obtained from the National Institute of Diabetes and Digestive and Kidney Diseases (NIDDK), or the clinician can use the professional Semmes-Weinstein filaments. Diabetic ulcers are evaluated and graded by the clinicians using different grading systems such as the Wagner classification system and the University of Texas classification system described herein.

Arterial ulcer: arterial leg ulcers refers to ulcers caused by poor blood supply to the legs, and is most often due to atherosclerosis. One embodiment of the present invention concerns the treatment of arterial ulcers. In particular arterial ulcers on subjects with an ankle brachial index less than 0.95, preferably from 0.95 to 0.2, preferably 0.8 to 0.6, preferably 0.8 to 0.2, preferably 0.6 to 0.2, preferably 0.5 to 0.2 as obtained using the Doppler test.

The terms "anastomosis" or "anastomotic" means a surgical connection between 2 structures. It usually means a connection that is created between tubular structures, such as blood vessels or loops of intestine. For example, when a segment of intestine is surgically removed, the 2 remaining ends are sewn or stapled together

(anastomosed), and the procedure is referred to as an intestinal anastomosis. One embodiment of the invention relates to the treatment of lesions in the mucosa, in particular ulcers due to gastro-intestinal anastomosis described herein. In a preferred embodiment the condition is an anastomotic ulcer(s) in the gastro-intestinal tract. The anastomosis may impair the blood supply to the tissue causing intestinal ischemia, infarction and ulceration of the tissue. Intestinal ischemia may be measured as described in Sheridan WG, Lowndes RH, Young HL. Intraoperative tissue oximetry in the human gastrointestinal tract. Am J Surg 1990; 159: 314-19.

In a preferred embodiment of the invention the condition is chronic and/or ischemic. In another preferred embodiment the condition is not infected or the subject is not infected with Mycobacterium tuberculosis, Mycobacterium bovis or Mycobacterium kansasii. In yet another preferred embodiment of the present invention the condition is not gangrene.

In one embodiment, the present invention relates to the treatment of diabetic ulcers. In particular the invention relates to the treatment of a diabetic ulcer(s) selected from at least one group of ulcers ranked according to the Wagner's classification system as Grad 1-C, 1-D, 2-C, 2-D, 3-C, and 3-D ulcers. Using the Texas Health Science Center San Antonio classification the ulcers subject for treatment according to the invention include the group of Grad 0-A, I-A, II-A, III-A ulcers, and the group of Grad 0-B, I-B, II-B, III-B, and the group of Grad 0-C, I-C, II-C, III-C, and the group of Grad 0-D, I-D, II-D, III-D. In yet another preferred embodiment the oxygen tension at the area of the condition is 50 mmHg or lower, such as 40 mmHg or lower or 30 mmHg or lower.

In another embodiment, the present invention relates to the treatment of pressure ulcers. In particular the invention relates to the treatment of a pressure ulcer(s) selected from at least one group of ulcers ranked according to the Wagner's classification system as Grad 1-C, 1-D, 2-C, 2-D, 3-C, and 3-D ulcers. Using the Texas Health Science Center San Antonio classification the ulcers subject for treatment according to the invention include the group of Grad 0-A, I-A, II-A, III-A ulcers, and the group of Grad 0-B, I-B, II-B, III-B, and the group of Grad 0-C, I-C, II-C, III-C, and the group of Grad 0-D, I-D, II-D, III-D. In yet another preferred embodiment the oxygen tension at the area of the condition is 50 mmHg or lower, such as 40 mmHg or lower or 30 mmHg or lower.

In one embodiment of the invention, the condition is classified according to the Wagner classification system of wounds as Grad 1-C, 1-D, 2-C, 2-D, 3-C or 3-D. In another embodiment the condition is classified according to the University of Texas classification system as Grad 0-A, I-A, II-A and III-A. In another embodiment the condition is classified according to the University of Texas classification system as Grad 0-B, I-B, II-B and III-B. In a preferred embodiment, the condition is classified according to the University of Texas classification system as Grad 0-C, I-C, II-C and III-C. In another preferred embodiment, the condition is classified according to the University of Texas classification system as Grad 0-D, I-D, II-D and III-D. In a preferred embodiment of the invention the oxygen tension at the tissue area is 50 mmHg or lower, such as 40 mmHg or lower, or 30 mmHg or lower.

In a preferred embodiment of the invention, the condition is not due to an infection, such as e.g. not due to an organisms of the genus Mycobacterium, specifically M. tuberculosis, M. bovis and M. kansasii. Especially, in a preferred embodiment of the invention, the condition is not due to an infection by Mycobacterium tuberculosis. However, as is evident from the various classification systems mentioned herein, a wound or ulcer, such as e.g. a chronic wound or ulcer, may during a prolonged, or even interrupted, healing process subsequently be infected by various micro-organisms. In some instances, it may be necessary to supplement the treatment according to the invention with a further active substance, such as e.g. a bactericidal agent, so as to treat a possible infection concomitantly with the treatment of the wound, ulcer, lesion or infarct with compromised vascularisation.

### Further aspects of the invention

In a further aspect, the present invention relates to the use of isoniazid in the preparation of a pharmaceutical composition for treating (or preventing) conditions described herein.

In this further aspect, the present invention relates to the use of isoniazid in the preparation of a pharmaceutical composition for treating (or preventing) a condition selected from the group consisting of cutaneous lesions, wounds, ulcers and infarcts in a tissue area with compromised vascularisation.

Heteroaryl means a monovalent aromatic cyclic radical having one to three rings, of four to eight atoms per ring, incorporating one or two heteroatoms (chosen from nitrogen, oxygen, or sulphur) within the ring which can optionally be substituted with one or two substituents selected from the group consisting of hydroxy, cyano, lower alkyl, lower alkoxy, lower haloalkoxy, alkylthio, halo, haloalkyl, hydroxyalkyl, nitro, alkoxycarbonyl, amino, alkylamino, alkylsulfonyl, arylsulfonyl, alkylaminosulfonyl, arylaminosulfonyl, alkylsulfonylamino, arylsulfonylamino, alkylaminocarbonyl, arylaminocarbonyl, alkylcarbonlamino and arylcarbonylamino.

Moieties of a particular compound cover group(s) or part(s) of said particular compound.

Substituted lower alkyl means a lower alkyl having one to three substituents selected from the group consisting of hydroxyl, alkoxy; amino, amido, carboxyl, acyl, halogen, cyano, nitro and thiol.

Other embodiments described herein apply *mutatis muntandis* to this further aspect of the present invention.

### Use in treatment

In a second aspect, the present invention relates to use of isoniazid in treating a condition selected from wounds, ulcers or infarcts in a tissue area with compromised vascularisation in an animal, such as a human subject, said use comprises administering to said human subject an effective dosage of isoniazid

or an effective dosage of pharmaceutically acceptable salts or solvates thereof.

In a preferred embodiment of the use according to the invention, the condition is selected from wounds, ulcers or infarcts in a tissue area with compromised vascularisation in a human subject.

In a preferred embodiment the condition is selected from the group consisting of pressure ulcers, diabetic ulcers, arterial ulcers and anastomotic ulcers. In a more preferred embodiment of the method, the tissue area with compromised vascularisation is ischemic. In another embodiment the condition is chronic. However, the embodiments described herein above for the first aspect of the invention, also apply for this aspect of the invention.

In the use according to the invention, isoniazid may be administered topically in a daily dosage of at least 0.01 mg/cm² wound area, such as from 0.1 mg/cm² to 100 mg/cm², from 0.5 mg/cm² to 100 mg/cm², preferably from 0.5 mg/cm² to 50 mg/cm², from 0.5 mg/cm² to 30 mg/cm², for example, 0.5 mg/cm² to 20 mg/cm², more preferred from 0.5 mg/cm² to 10 mg/cm², for example 1 mg/cm² to 7 mg/cm², 1 mg/cm² to 5 mg/cm², in particular 2 mg/cm² to 4 mg/cm², most preferred 3 mg/cm² wound area. This is for instance the case when the compounds are administered topically. In one embodiment of the invention isoniazid is administered topically in a daily dosage in a range of from about 0.01 mg/cm² to about 100 mg/cm² wound area, such as from 0.01 mg/cm² to 50 mg/cm², from 0.01 mg/cm² to 30 mg/cm², from 0.01 mg/cm² to 20 mg/cm², from 0.01 mg/cm² to 10 mg/cm², from 0.01 mg/cm² to 5 mg/cm², and from 0.01 mg/cm² to 2 mg/cm².

In a preferred embodiment of the use according to the invention, the daily dosage of isoniazid is in a range of from about 0.1 mg/kg to about 50 mg/kg bodyweight, such as from about 0.1 mg/kg to about 30 mg/kg, from about 0.5 mg/kg to about 20 mg/kg, from about 0.5 mg/kg to about 10 mg/kg, from about 1 mg/kg to about 7 mg/kg, from about 1 mg/kg to about 5 mg/kg, from about 2 mg/kg to about 5 mg/kg, and from about 2 mg/kg to about 4 mg/kg bodyweight. This is especially relevant in connection with oral administration.

In one embodiment of the use according to the invention a further active substance is administered. Preferably this further active substance is one or more antibacterial agents.

The features mentioned above for the use of a compound, or pharmaceutically acceptable salts, solvates or prodrugs thereof, for the preparation of a farmaceutical composition, apply *mutatis mutandis* for the use in treatment according to the present invention.

### Pharmaceutical compositions

For use in the present invention the compounds may be administered alone, but will generally be administered in admixture with suitable pharmaceutical excipients, diluents or carriers selected with regard to the intended route of administration and standard pharmaceutical practice. Accordingly, a pharmaceutical composition may further comprise one or more pharmaceutically acceptable excipients, diluents or carriers.

The compounds according to the invention may be formulated in any suitable manner according to the route of administration. The route of administration may be selected from the group of but not restricted to oral, topical, transdermal, rectal, nasal, pulmonal administration, it is however often preferred that the compound is administered orally or topically. In one embodiment of the invention, the pharmaceutical composition is formulated for systemic or local administration, such as e.g. oral, topical, transdermal, rectal, nasal, pulmonal, or parenteral administration. In a preferred embodiment, the pharmaceutical composition is formulated for oral administration.

In the treatment of some conditions other routes than systemic administration of the compound are preferable. Systemic compounds may not be delivered to an ischemic tissue due to the poor circulation, or systemic administration may be associated with adverse (system-wide) side effects of the compound, which are absent or reduced using other routes of administration, such as, but not restricted to, topical administration. Accordingly, when non-systemic administration, such as e.g., topical administration, is applied the daily dosage may be higher due to a potential reduction in systemic side effects.

Pharmaceutical compositions according to the invention comprise an effective dosage of at least one compound according to the invention and at least one pharmaceutical acceptable additive. The pharmaceutical acceptable additives may be any conventionally used pharmaceutical acceptable additive, which should be selected according to the specific formulation, intended administration route etc. For example the pharmaceutical acceptable additives may be any of the additives mentioned in Nema et al, 1997. Furthermore, the pharmaceutical acceptable additive may be any accepted additive from FDA's "inactive ingredients list", which for example is available on the internet address http://www.fda.gov/cder/drug/iig/default.htm.

In one embodiment of the present invention, a composition is formulated for topical application on a local, superficial and restricted bodily surface area comprising at least one pharmaceutically acceptable additive and at least one therapeutic compound according to the invention. An embodiment of the present invention concerns the use of compounds formulated for topical application. The pharmaceutical composition may take any forms or be impregnated in wound dressings known in the art of wound healing such as but not necessarily restricted to a cream, ointment, gel, solution, lotion, liniment, viscous emulsion, powder, paste, beads, a film dressing such as polyurethane film (Tegaderm), a foam dressing such as a polyethane or polyurethane foam dressing (such as the highly absorbent hydrophilic foam products Allevyn and Allevyn Adhesive, an island dressing Tielle, and Lyfoam), a hydrocolloid dressing (e.g Comfeel), a hydrogel (such as such as Carrington gel, Intrasite Gel, Granugel, Nu-gel), alginate (such as Sorbsan , Tegagen, Kaltostat or other gel forming polysaccharide dressings such as Aquacel), gauze, paraffin gauze, hypertonic-saline-gauze, wet-dry-saline gauze, continuously-moist-saline gauze, expanding dressings (e.g Dermasorb spiral wound dressing, Cutinova ), or Silver nanotech (e.g. Aquacel Ag), or dressings according to US 6,355,858. The selection of dressing depends on the specific condition, grade, description, characteristics and bacterial profile.

In one embodiment the composition comprised within a plaster, occlusive plaster, patch, dressing, or transdermal patch. In one embodiment of the present invention the formulation of said pharmaceutical composition is selected from the group of cream, ointment, gel, solution, lotion, liniment, viscous emulsion, powder, paste, beads, film dressing, foam, alginate, hydrocolloid dressing, and hydrogel. In a preferred embodiment of the invention the pharmaceutical composition is a cream, ointment, gel, solution, lotion, liniment, viscous emulsion, powder, paste, film dressing, foam, hydrocolloid dressing, or hydrogel. In another embodiment the pharmaceutical composition is formulated and integrated in a device selected from the group of plaster, occlusive plaster, patch, dressing, transdermal patch gauze, paraffin gauze, hypertonic-saline-gauze, wet-dry-saline gauze, continuously-moist-saline gauze, expanding dressings, Silver nanotech, or dressings according to US 6,355,858. In a preferred embodiment the pharmaceutical composition is integrated in a device selected from the group of plaster, occlusive plaster, patch, dressing, transdermal patch gauze, paraffin gauze, hypertonic-saline-gauze, wet-dry-saline gauze, continuously-moist-saline gauze, and expanding dressings.

In one embodiment a topically administrable composition of the compounds of the present invention is in the form of a hydrogel. Polymers for use in the compositions of the present invention include, but not limited to, the following hydrogels: cellulose derivatives such as carboxymethyl cellulose, hydroxymethyl cellulose, hydroxypropyl cellulose, hydroxyethyl cellulose, natural gums and the like, copolymers of polyethylene-polyoxypropylene diol block, polyacrylic acids, poly(ethylene oxide), poly(ethylene glycol),polyvinyl alcohol), poly (vinyl pyrrolidine), poly(acrylic acid), poly(hydroxy ethyl methacrylate), and chitosan and mixtures thereof.

Pharmaceutical compositions containing a compound of the present invention may be prepared by conventional techniques, e.g. as described in Remington: The Science and Practice of Pharmacy 1995, edited by E. W. Martin, Mack Publishing Company, 19th edition, Easton, Pa. The compositions may appear in conventional forms, for example capsules, tablets, aerosols, solutions, suspensions or topical applications.

For example, the compounds to be used in accordance with the invention can be administered orally, buccally or sublingually in the form of tablets, capsules (including soft gel capsules), ovules, elixirs, solutions or suspensions, which may contain flavouring or colouring agents, for immediate-, delayed-, modified-, sustained-, dual-, controlled-release or pulsatile delivery applications. The compounds of the invention may also be administered via fast dispersing or fast dissolving dosage forms. In a preferred embodiment of the invention, the pharmaceutical composition is a tablet, capsule, ovule, elixir, solution or suspension.

Tablets may contain excipients such as microcrystalline cellulose, lactose, sodium citrate, calcium carbonate, dibasic calcium phosphate, glycine, and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatine and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols.

Modified release and pulsatile release dosage forms may contain excipients such as those detailed for immediate release dosage forms together with additional excipients that act as release rate modifiers, these being coated on and/or included in the body of the device. Release rate modifiers include, but are not exclusively limited to, hydroxypropylmethyl cellulose, methyl cellulose, sodium carboxymethylcellulose, ethyl cellulose, cellulose acetate, polyethylene oxide, Xanthan gum, Carbomer, ammonio methacrylate copolymer, hydrogenated castor oil, carnauba wax, paraffin wax, cellulose acetate phthalate, hydroxypropylmethyl cellulose phthalate, methacrylic acid copolymer and mixtures thereof. Modified release and pulsatile release dosage forms may contain one or a combination of release rate modifying excipients. Release rate modifying excipients may be present both within the dosage form i.e. within the matrix, and/or on the dosage form, i.e. upon the surface or coating. Fast dispersing or dissolving dosage formulations (FDDFs) may contain the following ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol, xylitol. The terms dispersing or dissolving as used herein to describe FDDFs are dependent upon the solubility of the drug substance used i.e. where the drug substance is insoluble a fast dispersing dosage form can be prepared and where the drug substance is soluble a fast dissolving dosage form can be prepared.

For aqueous suspensions and/or elixirs, the compounds of the invention, or the pharmaceutically acceptable salts or solvates thereof, may be combined with various sweetening or flavouring agents, colouring matter or dyes, with emulsifying and/or suspending agents and with diluents such as water, ethanol, propylene glycol and glycerin, and combinations thereof.

The compounds for use in accordance with the invention can also be administered parenterally, for example, intravenously, intra-arterially, intraperitoneally, intrathecally, intraventricularly, intraurethrally, intramuscularly or subcutaneously, or they may be administered by infusion techniques. For such parenteral administration medicaments are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

The substances for use in accordance with the invention can also be administered intranasally or by inhalation and are conveniently delivered in the form of a dry powder inhaler or an aerosol spray presentation from a pressurised container, pump, spray or nebulizer with the use of a suitable propellant, e.g. dichlorodifluoromethane, trichlorofluoromethane, dichlorotetra- fluoro-ethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray or nebulizer may contain a solution or suspension of the active substance; e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of substance for use in accordance with the invention and a suitable powder base such as lactose or starch. The substances for use in accordance with the invention may also be formulated for delivery via an atomiser. Formulations for atomiser devices may contain the following ingredients as solubilisers, emulsifiers or suspending agents: water, ethanol, glycerol, propylene glycol, low molecular weight polyethylene glycols, sodium chloride, fluorocarbons, polyethylene glycol ethers, sorbitan trioleate, oleic acid.

Alternatively, the compounds for use in accordance with the invention can be administered by the rectal or topical route. This may be in the form of a suppository, or by topical application in the form of a gel, hydrogel, lotion; solution, cream, ointment, dusting powder or skin patch. For application topically to the skin, the substances can be formulated as a suitable ointment containing the active compound suspended or dissolved in, for example, a mixture with one or more of the following: mineral oil, liquid petrolatum, white petrolatum, propylene glycol, polyoxyethylene, polyoxypropylene compound, emulsifying wax and water. Alternatively, the substances can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters, wax, cetearyl alcohol, 2-octyldodecanol, benzyl alcohol and water.

The compounds for use in accordance with of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

### Dosage

The compound(s) according to the invention is administrated to a subject in need thereof. "Subject" includes living organisms such as animals, e.g. humans, monkeys, cows, sheep, horses, pigs, cattle, goats, dogs, cats, mice, rats, and transgenic species thereof. In a preferred embodiment, the subject is a human being.

The compound(s) according to the invention may be administered in any suitable manner according to the formulation thereof, at dosages and for periods of time effective to treat the condition in the subject.

An effective amount of the therapeutic compound necessary to achieve a therapeutic effect may vary according to factors such as the age, sex, and weight of the subject, and the ability of the therapeutic compound to treat the foreign agents in the subject. Dosage regimens can be adjusted to provide the optimum therapeutic response. For example, several divided doses may be administered daily or the dose may be proportionally reduced as indicated by the exigencies of the therapeutic situation.

In the context of the present invention, the term "daily dosage" is meant to describe the daily dosage required for e.g. an average human subject having a weight of about 65 to about 70 kg. The skilled person will readily be able to determine the dosage levels required for a subject whose weight falls outside the average range, such as children and the elderly. The daily dosage may optionally be administered as a single dose or be divided in two or more doses, such as e.g. two, three, or four, for administration at different times during the day. The physician will in any event determine the actual dosage which will be most suitable for any particular patient and it will vary with the age, weight and response of the particular patient. The below dosages are, of course only exemplary of the average case and there may be instances where higher or lower doses are merited and such are within the scope of the invention.

In one embodiment of the invention, the daily dosage of the compound is at least 0.01 mg/kg bodyweight, such as from 0.1 mg/kg to 100 mg/kg, from 0.5 mg/kg to 100 mg/kg, preferably from 0.5 mg/kg to 50 mg/kg, from 0.5 mg/kg to 30 mg/kg, for example, 0.5 mg/kg to 20 mg/kg, more preferred from 0.5 mg/kg to 10 mg/kg, for example 1 mg/kg to 7 mg/kg, 1 mg/kg to 5 mg/kg, in particular 2 mg/kg to 4 mg/kg, most preferred 3 mg/kg bodyweight. In a preferred embodiment of the invention, the pharmaceutical composition is given in a daily dosage in a range of from about 0.1 mg/kg to about 50 mg/kg bodyweight, such as from about 0.1 mg/kg to about 30 mg/kg, from about 0.5 mg/kg to about 20 mg/kg, from about 0.5 mg/kg to about 10 mg/kg, from about 1 mg/kg to about 7 mg/kg, from about 1 mg/kg to about 5 mg/kg, from about 2 mg/kg to about 5 mg/kg, and from about 2 mg/kg to about 4 mg/kg bodyweight. These dosages are relevant for any administration form; however, they are especially relevant for systemic administration, such as e.g., oral administration.

In another embodiment of the invention, in relation to topical application of the compounds disclosed herein, the daily dosage of the compound is at least 0.01 mg/cm² wound area, such as from 0.1 mg/cm² to 100 mg/cm², from 0.5 mg/cm² to 100 mg/cm², preferably from 0.5 mg/cm² to 50 mg/cm², from 0.5 mg/cm² to 30 mg/cm², for example, 0.5 mg/cm² to 20 mg/cm², more preferred from 0.5 mg/cm² to 10 mg/cm², for example 1 mg/cm² to 7 mg/cm², 1 mg/cm² to 5 mg/cm², in particular 2 mg/cm² to 4 mg/cm², most preferred 3 mg/cm² wound area. In another embodiment of the invention the isoniazid or isoniazid analogs of formula (Ia) is administered topically in a daily dosage in a range of from about 0.01 mg/cm² to about 100 mg/cm² wound area, such as from 0.01 mg/cm² to 50 mg/cm², from 0.01 mg/cm² to 30 mg/cm², from 0.01 mg/cm² to 20 mg/cm², from 0.01 mg/cm² to 10 mg/cm², from 0.01 mg/cm² to 5 mg/cm², and from 0.01 mg/cm² to 2 mg/cm².

The present invention further relates to the use of the compounds in combination with at least one other compound and/or at least one other treatment. Accordingly, the compounds may be administered simultaneously, either as separate formulations or combined in a unit dosage form, or administered sequentially. The combination medicament may be formulated by co-formulating the compound according to the invention with another therapeutic agent for simultaneous administration. In another embodiment the combination medicament is formulated as two separate medicaments for either simultaneous or sequential administration. In another preferred embodiment, said composition comprises at least one second active ingredient, as described herein above. Said second active ingredient may be any second active ingredient as disclosed herein. Said second active ingredient may be an antibiotic for the treatment of conditions involving a bacterial infection. Another embodiment of the invention concerns the use of such a composition.

In a preferred embodiment of the invention the pharmaceutical composition further comprises one or more additional active substances. Such additional active substances may include, but are not limited to, antibacterial therapeutic agents, antifungal therapeutic agents, antimycobacterial therapeutic agents, non-steroidal antiinflammatory drugs (NSAIDS), and combinations thereof (e.g., an antibacterial therapeutic agent and an antifungal therapeutic agent).

In a more preferred embodiment of the invention, the pharmaceutical composition further comprises one or more antibacterial agents. Non-limiting examples of antibacterial, agents are beta-lactams, such as penicillins (e.g., penicillin G, oxacillin, ampicillin, nafcillin, ticarcillin, and amoxicillin), cephalosporins (e.g., cephalothin, cephalexin, cefazolin, cephradine, cephapirin, cefamandole, cefoxitin, cefoperazone, cefotaxime, and ceftriaxone), monbactams (e.g., aztreonam), beta-lactamase inhibitors (e.g., clavulanic acid, sulbactam, and tazobactam), and carbapenems (e.g., imipenem), as well as derivatives of such beta-lactams; polypeptides, such as bacitracin; aminoglycosides, such as neomycin, gentamicin, clindamycin, tobramycin, amikacin, netilmicin, and lincomycin; amino-glycosidic-type compounds, such as spectinomycin; macrolides, such as erythromycin, streptomycin, azithromycin, and clarithromycin; azoles such as metronidazole; mupirocin; azines, such as silver sulfadiazine; inhibitors of bacterial cell wall synthesis, such as vancomycin, teicoplanin, and fosfomycin; and combinations of two or more antibacterial agents.

In a more preferred embodiment of the invention, the pharmaceutical composition further comprises one or more antifungal agents. Examples of antifungal agents suitable for use in the present invention include, but are not limited to, azoles, such as miconazole, clotrimazole, ketoconazole, oxiconizole, econazole, sulconazole, fluconazole, and itraconazole; allylamines, such as naftifine and terbinafine; benzylamines, such as butenafine; polyenes, such as nystatin and amphotericin B; thiocarbonates, such as tolnaftate; sulfides, such as selenium sulfide; nitrogen-containing heterocycles, such as ciclopirox, and combinations of two or more antifungal agents.

In another preferred embodiment of the invention, the pharmaceutical composition further comprises a combination of one or more antibacterial agent and one or more antifungal agents.

By the term "separate administration" is meant an initial administration of a first compound/medicament followed by secondary administration of a compound/medicament. The order of administration of the compounds/medicaments is not significant, and the time interval with which the first administration is followed by the second administration is not determined.

The term "unit dosage form" as used herein refers to physically discrete units suitable as unitary dosages for human and animal individuals, each unit containing a predetermined quantity of a compound, alone or in combination with other agents, calculated in an amount sufficient to produce the desired effect in association with a pharmaceutically acceptable diluent, carrier, or vehicle. The specifications for the unit dosage forms of the present invention depend on the particular compound or compounds employed and the effect to be achieved, as well as the pharmacodynamics associated with each compound in the host. The dose administered should be an "effective amount" or an amount necessary to achieve an "effective level" in the individual patient.

Since the "affective level" is used as the preferred endpoint for dosing, the actual dose and schedule can vary, depending on inter-individual differences in pharmacokinetics, drug distribution, and metabolism. The "effective level" can be defined, for example, as the blood or tissue level desired in the individual that corresponds to a concentration of one or more compounds according to the invention. Also, the effective level is depending on the therapeutic agent in question, and in particular on the concentration of the effective level in question.

### Examples

### Example 1

### Assessing the effects of isoniazid on non-ischemic and ischemic cutaneous wound healing using a validated rat model.

### Materials and methods

### Animals and wound model

Male Sprague-Dawley rats (Charles River, Sulzfeld, Germany), weighing from 341 to 385 gram (363 ± 11 gram, mean ± SD), were fasted but allowed tap water ad libitum for 18 hours before surgical and experimental procedures. The institutional guidelines for care and use of laboratory animals were followed. Rats were anesthetized by intraperitoneal (i.p.) injection of 100 mg/kg ketamine (Parke-Davis, Berlin, Germany) and 15 mg/kg xylazin (Bayer, Leverkusen, Germany). A standardized pedunculated dorsal ischemic skin flap (3 cm x 7 cm) was lifted, replaced, and fixed with metal clips. Two full-thickness wounds were made using punch biopsies (8 mm diameter), one in the ischemic flap region and one non-ischemic outside the flap in the neck.

### Treatments

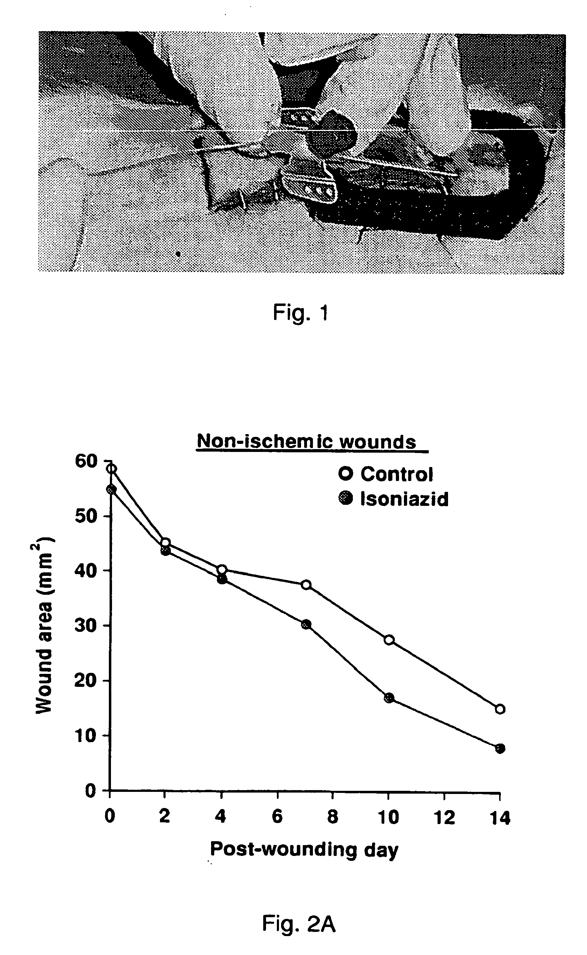

Isoniazid (13377; Sigma-Aldrich, St. Louis, MO, USA) dissolved in sterile isotonic saline (0.9% NaCl) at 1.0 mg/ml was administered b.i.d. at 10 ml/kg i.p. (10 mg/kg) to a group of 10 rats. To the control group, comprising 10 rats, 10 ml/kg of sterile isotonic saline was administered. The compounds were masked to the investigators. The first dose was given directly after wounding. Wounds were traced (outer margins) on OpSite* Flexigrid* (Smith & Nephew) 30 minutes after wounding day 0 and after dressing (Hydrosorb, Hartmann) removal and wound cleansing on subsequent days (Table I). On post-wounding day 2, intracutaneous oxygen tensions were measured 1 hour after administration of compounds with Licox® (Integra LifeSciences Corporation) Clark type probe under full anesthesia. The Licox® probe was positioned at the wound edges using a Venflon catheter (Fig. 1).

**Table I. Summarv of the procedures**

| Procedure | Day | | | | | | | | | | | | | | |
|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 |
| Wounding | X | | | | | | | | | | | | | | |
| Compound | X | X | X | X | X | X | X | X | X | X | X | X | X | X | |
| Dressing change | X | | X | | X | | | X | | | X | | | | |
| Wound tracing | X | | X | | X | | | X | | | X | | | | X |
| pₜᵢO₂ | | | X | | | | | | | | | | | | |

### Statistical analyses

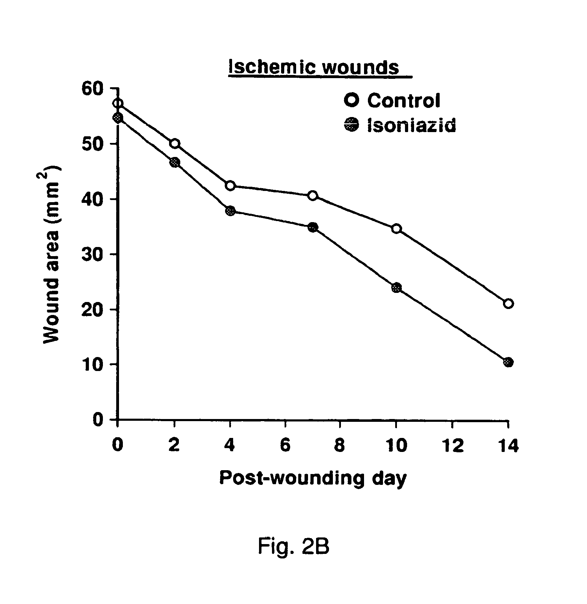

Results are expressed as mean ± SD or mean ± SEM. Wound healing differences among the two groups were calculated as the area under the wound healing curves (Fig. 2A+B) with correction according to Bonferroni (p = 0.035). Licox® results were analyzed by the Wilcoxon test.

### Results

### Tolerability

The compounds were generally well-tolerated. One rat in the control group died on post-wounding day 11, and one rat in the isoniazid group 5 days after operation. Body weights did not differ significantly among the three groups either before operation or after 14 days of treatment (Table II).

**Table II. Body weights (gram) of the rats before operation, and after being killed on post-wounding day 14 (Mean ± SD)**

| Time | Group | |
|---|---|---|
| | Control | Isoniazid |
| Before operation | 362 ± 9 | 360 ± 8 |
| n | 10 | 10 |
| Postoperative day 14 | 347 ± 13 | 341 ± 12 |
| n | 9 | 9 |

### Intracutaneous oxygen tension measurements

Ischemic wound conditions in the skin flap were verified by Licox® measurements. In the control group, ptiO₂ at the edges of non-ischemic wounds was 50.6 ± 7.6 mm Hg (n = 8) compared to 25.6 ± 10.2 mm Hg (n = 9) of ischemic wounds.

### Wound healing

As anticipated, healing was delayed of the ischemic wounds (Fig. 2B)compared with the non-ischemic wounds in the control group (Fig. 2A). Isoniazid treatment promoted healing of the ischemic wounds compared with the control (p = 0.014)

### Conclusions

Isoniazid stimulated ischemic wound healing.

### Example 2

### Clinical study of the effect of isoniazid on ischemic ulcers

The clinical study is a randomised, double blind, placebo-controlled study with parallel groups designed to investigate the effect of isoniazid on healing of ischemic leg ulcers. In this study 20 subject with ischemic leg ulcers (systolic toe pressure: < 40 mmHg) will be included.

The treatment period is 4 weeks, during which the subjects either will receive isoniazid (150 mg twice daily, oral) or placebo (twice daily, oral). All subjects will at the same time receive pyridoxine (20 mg daily) to prevent vitamin B₆ deficiency due to the treatment with isoniazid. The patients will furthermore receive conventional treatment with paraffin gaze:

The treatment will primarily be evaluated by the percentage change to the wound area at 8 weeks after treatment start. The progress of the wound healing will be registered after 0, 2, 4 and 8 weeks, or after shorter periods of time if the wound is healed prior to the end of the 8 week period. However, the treatment will furthermore be evaluated by the pain intensity as established by a visual analog scale, together with the occurrence of suspected unexpected serious adverse reactions (SUSAR) serious adverse events (SAE) or side-effects. Table III shows an overview of the procedures.

**Table III. Overview of procedures**

| Procedure | Week | | | | |
|---|---|---|---|---|---|
| | -1 | 0 | 2 | 4 | 8 |
| Anamnesis | X | | | | |
| Vascular investigation | X | | | | |
| Tobacco consumption | X | | | | |
| Clinical assessment | X | X | X | X | X |
| Measurement of wound area¹ | X | X | X | X | X |
| Registration of pain | X | X | X | X | X |
| Serology² | X | X | X | X | X |
| SUSAR/SAE | | X | X | X | X |
| Photography of wounds | | X | X | X | X |
| Inclusion and written consent | | X | | | |
| Randomisation | | X | | | |

| | | | | | |
|---|---|---|---|---|---|
| ¹ By drawing the wound area on clear plastic (Opsite Flexigrid) with subsequent analysis (Scion Image). ² Hb (8.0-11.0 mmmol/l) CRP, Creatinin and ASAT (15-35 U/l) | | | | | |

### Inclusion criteria

- age ≥ 18 years
- Subjects with ischemic leg ulcers, where blood vessel surgery is not possible.
- systolic toe pressure: < 40 mmHg
- Duration of wound > 3 months
- Wound area 1-20 cm²
- Written consent

### Exclusion criteria

- B-Hb (haemoglobin) < 6 mmol/l
- ASAT (aspartate aminotransferase) > 50 U/I
- Diabetes Mellitus
- Use of more than 10 mg of prednisolone daily for the last 30 days
- Use of cytotoxic agents during the last 3 months
- Use of rifampicin, phenytoin, carbamazepin, theophyllin, benzodiazepines (diazepam, triazolam), stavudin and/or valproate
- Anamnesis with alcohol abuse
- Hereditary galactose intolerans
- Allergy towards isoniazid or one or more of the excipients (magnesium stearate, polyvidon, talc, lactose and starch)
- Gangrene
- Participation in clinical studies, within the last 7 days
- Pregnant or breast-feading women
- Fertile women, not using contraception

Routine treatment and treatment with antibiotics in cases with infection during the study period is allowed. Compliance is assessed by the use of a subject diary, with registration of time of administration of medicine.

## Claims

1. Isoniazid, or pharmaceutically acceptable salts or solvates thereof, for use in treating a condition selected from the group consisting of cutaneous lesions, wounds, ulcers and infarcts in a tissue area with compromised vascularisation, in a human subject.

2. The isoniazid according to any of the preceding claims for use in treating a condition, where the condition is selected from the group consisting of pressure ulcers, diabetic ulcers, arterial ulcers and anastomotic ulcers.

3. The isoniazid according to any of the preceding claims for use in treating a condition, wherein the condition is classified according to the Wagner classification system of wounds as Grad 1-C, 1-D, 2-C, 2-D, 3-C or 3-D.

4. The isoniazid according to any of the preceding claims for use in treating a condition, wherein the condition is classified according to the University of Texas classification system as Grad 0-C, I-C, II-C and III-C.

5. The isoniazid according to any of the preceding claims for use in treating a condition, wherein the tissue area with compromised vascularisation is ischemic.

6. The isoniazid according to any of the preceding claims for use in treating a condition, wherein the condition is chronic.

7. The isoniazid according to any of the preceding claims for use in treating a condition, wherein the condition is not due to an infection; o⁻ not due to an infection by Mycobacterium tuberculosis.

8. The isoniazid according to any of the preceding claims for use in treating a condition, wherein the condition is an ischemic ulcer(s).

9. The isoniazid according to any of the preceding claims for use in treating a condition, wherein the condition is a pressure ulcer(s).

10. The isoniazid according to any of the preceding claims for use in treating a condition, wherein the condition is a diabetic ulcer(s).

11. The isoniazid according to any of the preceding claims for use in treating a condition, wherein the condition is arterial ulcer(s).

12. A pharmaceutical composition comprising the isoniazid according to any of the preceding claims for use in treating a condition according to any of the preceding claims, wherein the pharmaceutical composition further comprises one or more pharmaceutically acceptable excipients, diluents or carriers.

13. The pharmaceutical composition according to claim 12, wherein the pharmaceutical composition is formulated for systemic or local administration, such as e.g. oral, topical, transdermal, rectal, nasal, pulmonal, or parenteral administration.

14. The pharmaceutical composition according to any of the claims 12-13, wherein the pharmaceutical composition is a cream, ointment, gel, solution, lotion, liniment, viscous emulsion, powder, paste, film dressing, foam, hydrocolloid dressing, or hydrogel.

15. The pharmaceutical composition according to any of the claims 12-14, wherein the pharmaceutical composition is integrated in a device selected from the group of plaster, occlusive plaster, patch, dressing, transdermal patch gauze, paraffin gauze, hypertonic-saline-gauze, wet-dry-saline gauze, continuously-moist-saline gauze, and expanding dressings.

16. The pharmaceutical composition according to any of the claims 12-15, wherein the pharmaceutical composition further comprise one or more additional active substances; preferably one or more antibacterial agents.

## Patentansprüche

1. Isoniazid, oder pharmazeutisch akzeptierbare Salze oder Solvate davon, für die Verwendung beim Behandeln eines Leidens, gewählt aus der Gruppe bestehend aus Hauterkrankungen, Wunden, Geschwüren und Infarkten in einem Gewebebereich mit beeinträchtigter Vaskularisation, bei einem Humanpatienten.

2. Isoniazid nach einem der vorhergehenden Ansprüche, zur Verwendung beim Behandeln eines Leidens, wobei das Leiden aus der Gruppe gewählt wird, die aus Druckgeschwüren, diabetischen Geschwüren, arteriellen Geschwüren und anastomotischen Geschwüren besteht.

3. Isoniazid nach einem der vorhergehenden Ansprüche, zur Verwendung beim Behandeln eines Leidens, wobei das Leiden nach dem Wagner Klassifikationssystem von Wunden als Gradient 1-C, 1-D, 2-C, 2-D, 3-C oder 3-D klassifiziert wird.

4. Isoniazid nach einem der vorhergehenden Ansprüche, zur Verwendung beim Behandeln eines Leidens, wobei das Leiden gemäß dem Klassifikationssystem der Universität von Texas als Gradient 0-C, I-C, II-C und III-C klassifiziert wird.

5. Isoniazid nach einem der vorhergehenden Ansprüche, zur Verwendung beim Behandeln eines Leidens, wobei der Gewebebereich mit beeinträchtigter Vaskularisation ischämisch ist.

6. Isoniazid nach einem der vorhergehenden Ansprüche, zur Verwendung beim Behandeln eines Leidens, wobei das Leiden chronisch ist.

7. Isoniazid nach einem der vorhergehenden Ansprüche, zur Verwendung beim Behandeln eines Leidens, wobei das Leiden nicht von einer Infektion stammt; oder nicht von einer Infektion durch Mycobakterium tuberculosis stammt.

8. Isoniazid nach einem der vorhergehenden Ansprüche, zur Verwendung beim Behandeln eines Leidens, wobei das Leiden ein ischämisches Geschwür ist.

9. Isoniazid nach einem der vorhergehenden Ansprüche, zur Verwendung beim Behandeln eines Leidens, wobei das Leiden ein Druckgeschwür ist.

10. Isoniazid nach einem der vorhergehenden Ansprüche, zur Verwendung beim Behandeln eines Leidens, wobei das Leiden ein diabetisches Geschwür ist.

11. Isoniazid nach einem der vorhergehenden Ansprüche, zur Verwendung beim Behandeln eines Leidens, wobei das Leiden ein arterielles Geschwür ist.

12. Pharmazeutische Zusammensetzung, welche Isoniazid nach einem der vorhergehenden Ansprüche aufweist, zur Verwendung bei der Behandlung eines Leidens nach einem der vorhergehenden Ansprüche, wobei die pharmazeutische Zusammensetzung weiterhin einen oder mehrere pharmazeutisch akzeptierbare Hilfsstoffe, Lösungsmittel oder Trägerstoffe aufweist.

13. Pharmazeutische Zusammensetzung nach Anspruch 12, wobei die pharmazeutische Zusammensetzung für systemische oder lokale Verabreichung formuliert ist, wie beispielsweise orale, topische, transdermale, rektale, nasale, pulmonale oder parenterale Verabreichung.

14. Pharmazeutische Zusammensetzung nach einem der Ansprüche 12 oder 13, wobei die pharmazeutische Zusammensetzung eine Creme, eine Salbe, ein Gel, eine Lösung, eine Lotion, ein Einreibemittel, eine Viskoseemulsion, ein Puder, eine Paste, ein Filmpflaster, ein Schaum, ein Hydrocolloidpflaster oder ein Hydrogel ist.

15. Pharmazeutische Zusammensetzung nach einem der Ansprüche 12 bis 14, wobei die pharmazeutische Zusammensetzung in eine Vorrichtung integriert ist, die aus der Gruppe: Wundpflaster, okklusive Pflaster, Pflaster, Verband, transdermales Pflastermull, Parafinmull, hypertonisches Kochsalzmull, Nass-Trocken-Kochsalzmull, Kontinuierlich-Feucht-Kochsalzmull und Dehnverbände.

16. Pharmazeutische Zusammensetzung nach einem der Ansprüche 12 bis 15, wobei die pharmazeutische Zusammensetzung eine oder mehrere zusätzliche aktive Substanzen aufweist; vorzugsweise einen oder mehrere antibakterielle Wirkstoffe.

## Revendications

1. Isoniazide, ou sels ou solvates pharmaceutiquement acceptables de celui-ci, destinés à être utilisés dans le traitement d'un état pathologique choisi dans le groupe comprenant des lésions cutanées, des blessures, des ulcères et des infarctus dans une zone de tissus dont la vascularisation est compromise, chez un sujet humain.

2. Isoniazide selon l'une quelconque des revendications précédentes, destiné à être utilisé dans le traitement d'un état pathologique, l'état pathologique étant choisi dans le groupe comprenant des escarres de décubitus, des ulcères diabétiques, des ulcères artériels et des ulcères anastomotiques.

3. Isoniazide selon l'une quelconque des revendications précédentes, destiné à être utilisé dans le traitement d'un état pathologique, l'état pathologique étant classifié selon le système de classification des plaies de Wagner comme étant de type 1-C, 1-D, 2-C, 2-D, 3-C ou 3-D.

4. Isoniazide selon l'une quelconque des revendications précédentes, destiné à être utilisé dans le traitement d'un état pathologique, l'état pathologique étant classifié selon le système de classification de l'Université du Texas comme étant de type 0-C, I-C, II-C et III-C.

5. Isoniazide selon l'une quelconque des revendications précédentes, destiné à être utilisé dans le traitement d'un état pathologique, la zone de tissus dont la vascularisation est compromise étant ischémique.

6. Isoniazide selon l'une quelconque des revendications précédentes, destiné à être utilisé dans le traitement d'un état pathologique, l'état pathologique étant chronique.

7. Isoniazide selon l'une quelconque des revendications précédentes, destiné à être utilisé dans le traitement d'un état pathologique, l'état pathologique n'étant pas dû à une infection ; ou n'étant pas dû à une infection par *Mycobacterium tuberculosis*.

8. Isoniazide selon l'une quelconque des revendications précédentes, destiné à être utilisé dans le traitement d'un état pathologique, l'état pathologique étant un ulcère ischémique.

9. Isoniazide selon l'une quelconque des revendications précédentes, destiné à être utilisé dans le traitement d'un état pathologique, l'état pathologique étant une escarre de décubitus.

10. Isoniazide selon l'une quelconque des revendications précédentes, destiné à être utilisé dans le traitement d'un état pathologique, l'état pathologique étant un ulcère diabétique.

11. Isoniazide selon l'une quelconque des revendications précédentes, destiné à être utilisé dans le traitement d'un état pathologique, l'état pathologique étant un ulcère artériel.

12. Composition pharmaceutique comprenant l'isoniazide selon l'une quelconque des revendications précédentes destiné à être utilisée dans le traitement d'un état pathologique selon l'une quelconque des revendications précédentes, la composition pharmaceutique comprenant en outre un ou plusieurs excipients, diluants ou transporteurs pharmaceutiquement acceptables.

13. Composition pharmaceutique selon la revendication 12, la composition pharmaceutique étant formulée pour une administration par voie systémique ou locale, telle que par exemple une administration par voie orale, topique, transdermique, rectale, nasale, pulmonaire, ou parentérale.

14. Composition pharmaceutique selon l'une quelconque des revendications 12 ou 13, la composition pharmaceutique étant une crème, un onguent, un gel, une solution, une lotion, un liniment, une émulsion visqueuse, une poudre, une pâte, un pansement sous forme de film, une mousse, un pansement sous forme d'hydrocolloïde, ou un hydrogel.

15. Composition pharmaceutique selon l'une quelconque des revendications 12 à 14, la composition pharmaceutique étant intégrée dans un dispositif choisi dans le groupe comprenant du plâtre, du plâtre occlusif, un timbre transdermique, un pansement, une gaze sous forme de timbre transdermique, une gaze de paraffine, une gaze humectée avec une solution saline hypertonique, une gaze semi-humide humectée avec une solution saline, une gaze humectée de manière continue avec une solution saline, et des pansements dilatables.

16. Composition pharmaceutique selon l'une quelconque des revendications 12 à 15, la composition pharmaceutique comprenant en outre une ou plusieurs substances actives supplémentaires ; de préférence un ou plusieurs agents antibactériens.
